# EUROPÄISCHE PATENTSCHRIFT

(11) **EP 4 097 072 B1**
(45) Veröffentlichungstag und Bekanntmachung des Hinweises auf die Patenterteilung: **13.03.2024**
(21) Anmeldenummer: 21702008.0
(22) Anmeldetag: 26.01.2021
(51) Int. Cl.: C07C 41/50, C07C 41/56, C07C 43/30

(54) **VERFAHREN ZUR HERSTELLUNG VON OXYMETHYLENETHER**
PROCESS FOR THE PRODUCTION OF OXYMETHYLENE ETHER
PROCÉDÉ DE PRODUCTION D'OXYMÉTHYLENÉTHER

(30) Priorität: 27.01.2020 EP 20153907
(43) Veröffentlichungstag der Anmeldung: 07.12.2022
(73) Patentinhaber: Friedrich-Alexander-Universität Erlangen-Nürnberg, 91054 Erlangen (DE); OxFA GmbH, 96110 Scheßlitz (DE)
(72) Erfinder: ALBERT, Jakob, 22523 Hamburg (DE); BUKOWSKI, Anna, 91052 Erlangen (DE); VOSS, Dorothea, 91056 Erlangen (DE)
(74) Vertreter: Dr. Gassner & Partner mbB
(86) Internationale Anmeldenummer: PCT/EP2021/051718
(87) Internationale Veröffentlichungsnummer: WO 2021/151871

(56) Entgegenhaltungen:
- WO-A1-2007/034264
- US-A1- 2005 154 226

## Beschreibung

Die Erfindung ist in den Ansprüchen definiert und betrifft ein Verfahren zur Erzeugung von Oxymethylenether (OME) der allgemeinen Formel CH₃O-(CH₂O)ₘ-CH₃ mit 1 ≤ m ≤ 10 in einer katalytischen Reaktion.

OMEs können als Additiv Dieselkraftstoff zugesetzt werden, um die Rußbildung in einem Dieselmotor zu vermindern.

Aus der DE 2 163 907 ist ein Verfahren zur Herstellung von Polyoxymethylendialkylethern bekannt, bei dem ein niedrigmolekularer Alkohol mit einer Formaldehydbildenden Substanz in Gegenwart eines sauren Katalysators umgesetzt wird.

Aus der DE 10 2005 027 702 A1 ist ein Verfahren zur Herstellung von Polyoxymethylendimethylethern aus Methanol und Formaldehyd bekannt. Dabei wird das dabei entstehende Gemisch, enthaltend Formaldehyd, Wasser, Methylenglykol, Polyoxymethylenglykole, Methanol, Hemiformale, Methylal und Polyoxymethylenglykoldimethylether durch Destillation aufgearbeitet. Nachteilig bei diesem Verfahren sowie dem aus der DE 2 163 907 bekannten Verfahren ist, dass ein komplexes Gemisch von Reaktionsprodukten entsteht und die Gewinnung von Polyoxymethylendimethylethern daraus mit erheblichem Aufwand verbunden ist.

Aus der WO 2006/045506 A1 ist ein Verfahren zur Herstellung von Polyoxymethylendimethylether bekannt, bei dem Methylal und Trioxan in Gegenwart eines sauren Katalysators umgesetzt werden. Das Verfahren zeichnet sich dadurch aus, dass die durch Methylal, Trioxan und/oder den Katalysator in das Reaktionsgemisch eingebrachte Wassermenge weniger als 1 Gew.-%, bezogen auf das Reaktionsgemisch, beträgt. Bei dem Verfahren wird zur Herstellung von längerkettigen Polyoxymethylendimethylethern Dimethoxymethan eingesetzt.

Aus der DE 10 2014 112 021 A1 ist ein Verfahren zur Herstellung von Oxymethylendialkylethern und deren direkte Verwendung als Kraftstoffadditive bekannt. Bei dem Verfahren wird ein Alkohol und/oder eine Carbonsäure mit einem Aldehyd und/oder einem Keton in Anwesenheit eines sauren Katalysators umgesetzt. Während der Reaktion oder anschließend wird durch Zugabe oder Entstehung eines Extraktionsmittels eine wässrige und eine organische Phase erzeugt und anschließend die organische Phase entnommen.

Allen oben genannten Verfahren auf Basis von Methanol ist gemein, dass sie eine geringe Selektivität besitzen und damit zu komplexen Produktgemischen führen, die zahlreiche unerwünschte Komponenten enthalten können.

Die US 2005/0154226 A1 offenbart ein Verfahren zur Oxidation einer gasförmigen Beschickung, die Methanol und/oder Dimethylether umfasst, um ein Produkt herzustellen, das hauptsächlich Dimethoxymethan oder hauptsächlich Methylformiat enthält. Dabei wird die Beschickung mit einem sauerstoffhaltigen Gas und einem geträgerten Heteropolysäure-Keggin-Katalysator, der Molybdän oder Molybdän und Vanadium enthält, in Kontakt gebracht. Unter den in den Ausführungsbeispielen genannten Bedingungen wurden keine homogenen Methanol-Reaktionen beobachtet.

Die WO 2007/034264 A1 betrifft Katalysatoren für eine Oxidation von Methanol, Ethanol, Propanol oder Butanol und ein Herstellungsverfahren für ein Teiloxidationsprodukt von Methanol, Ethanol, Propanol oder Butanol unter Verwendung der Katalysatoren. Bei dem Teiloxidationsprodukt kann es sich um ein Dialkoxymethan, wie Dimethoxymethan handeln. Der Katalysator kann ein Bulk-Katalysator oder ein geträgerter Katalysator sein. Bei dem Herstellungsverfahren wird Methanol, Ethanol, Propanol oder Butanol einer Dampfphasenkontaktoxidation mit einem molekularen Sauerstoff enthaltenden Gas in Gegenwart eines Katalysators unterworfen.

Aus Tang, Z. et al., ChemSusChem 2014, 7, Seiten 1557 bis 1567 ist eine durch Vanadyl-Kationen katalysierte Umsetzung von Zellulose zu Ameisensäure und Milchsäure bekannt. Insbesondere wird die Verwendung von VOSO₄ als Katalysator zur Umsetzung von Glucose zu Ameisensäure und zu Milchsäure offenbart. Durch die Bildung von CO₂ bei dieser Umsetzung ist die Ausbeute von Ameisensäure auf etwas über 50% beschränkt. Es wurde jedoch gefunden, dass das Zusetzen von Methanol oder Ethanol zu dem Reaktionssystem die Bildung von CO₂ während der Umsetzung von Glucose unter aeroben Bedingungen unterdrückt und die Ausbeute an Ameisensäure dadurch auf 70% bis 75% erhöht werden konnte.

Aufgabe der vorliegenden Erfindung ist es, ein alternatives Verfahren zur Herstellung von Oxymethylenether anzugeben. Insbesondere soll das Verfahren Oxymethylenether mit hoher Selektivität ohne eine große Zahl unerwünschter Nebenprodukte liefern.

Erfindungsgemäß wird die Aufgabe durch die Merkmale des Patentanspruchs 1 gelöst. Zweckmäßige Ausgestaltungen ergeben sich aus den Merkmalen der Patentansprüche 2 bis 15.

Erfindungsgemäß ist ein Verfahren zur Erzeugung von Oxymethylenether der allgemeinen Formel CH₃O-(CH₂O)ₘ-CH₃ in einem Flüssigphasenprozess vorgesehen, wobei 1 ≤ m ≤ 10, wobei in einer katalytischen Reaktion, insbesondere ausschließlich, molekularer Sauerstoff oder ein Sauerstoff enthaltendes Oxidationsmittel und Methanol, Formaldehyd und/oder Methylformiat in einer Lösung als Edukte eingesetzt und mittels einer Vanadium in der Oxidationsstufe +IV oder +V enthaltenden Vanadium-Sauerstoff-Verbindung oder eines Salzes davon als Katalysator in der Lösung umgesetzt werden, wobei der bei der katalytischen Reaktion reduzierte Katalysator durch Oxidation mittels dem molekularen Sauerstoff oder dem Sauerstoff enthaltenden und diesen Sauerstoff an den reduzierten Katalysator abgebenden Oxidationsmittel wieder in seinen Ausgangszustand versetzt wird. Der Katalysator liegt dabei im Allgemeinen in gelöster Form in der Lösung vor.

Der dabei erzeugte Oxymethylenether kann, insbesondere durch eine Extraktion oder mittels eines sonstigen bekannten Trennverfahrens, insbesondere unter Verwendung einer semipermeablen Membran, aus der Lösung abgesondert werden.

Bei einer Ausgestaltung dieses Verfahrens werden in der katalytischen Reaktion, insbesondere ausschließlich, der molekulare Sauerstoff oder das Sauerstoff enthaltendes Oxidationsmittel und Methanol in der Lösung als Edukte eingesetzt.

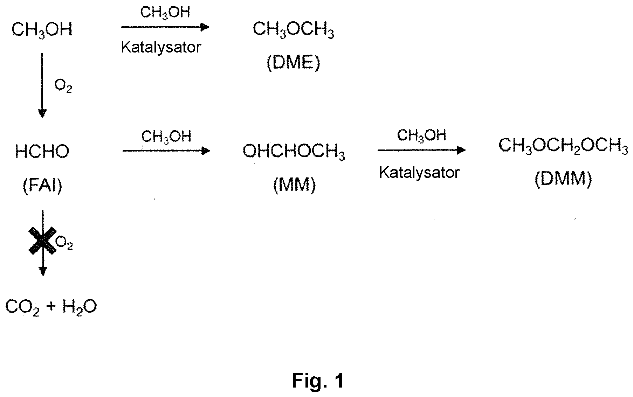

Die Erfinder haben festgestellt, dass durch die Verwendung von Methanol, Formaldehyd und/oder Methylformiat und molekularem Sauerstoff oder einem Sauerstoff enthaltenden Oxidationsmittel als Edukte direkt Dimethoxymethan, d. h. CH₃O-(CH₂O)ₘ-CH₃ mit m = 1, in flüssiger Phase erzeugt werden kann, selbst wenn ausschließlich diese Edukte verwendet werden. Die Erfinder nehmen an, dass bei der Umsetzung von Methanol durch teilweise partielle Oxidation des Methanols in-situ Formaldehyd (FAI) entsteht. Weiterhin nehmen sie an, dass das Formaldehyd anschließend mit weiterem Methanol zu Methoxymethanol (MM) und dieses in einem letzten Reaktionsschritt mit weiterem Methanol in Gegenwart des Katalysators zu Dimethoxymethan (DMM) reagiert. Eine vollständige Oxidation des Formaldehyds zu CO₂ und H₂O erfolgt überraschenderweise nicht oder zumindest nicht in wesentlichem Umfang. Als Nebenprodukt entsteht lediglich Dimethylether (DME), wobei das Verhältnis von DMM zu DME durch Wahl einer verhältnismäßig niedrigen Reaktionstemperatur, beispielsweise im Bereich zwischen 70 °C und 90 °C, deutlich zum DMM hin verschoben wird. Ein entsprechendes Reaktionsschema ist in Fig. 1 dargestellt. Das DMM kann durch eine Extraktion oder mittels eines sonstigen bekannten Trennverfahrens, insbesondere unter Verwendung einer semipermeablen Membran, aus der Lösung abgesondert werden.

Der Katalysator ist ein Polyoxometallat-Ion der allgemeinen Formel [PMoₓV_{y}O₄₀]ⁿ⁻, wobei 6 ≤ x ≤ 11, 1 ≤ y ≤ 6 und x + y = 12, [WₓV_{y}O₁₉]ⁿ⁻, wobei x + y = 6, 3 ≤ x ≤ 5 und 1 ≤ y ≤ 3 oder [P₂WₓV_{y}O₆₂]ⁿ⁻, wobei x + y = 18, 12 ≤ x ≤ 17 und 1 ≤ y ≤ 6 oder ein VO²⁺ enthaltendes Salz, insbesondere VOSO₄, oder ein [VO₃]⁻ enthaltendes Salz, insbesondere NH₄VO₃, wobei n, x und y jeweils eine ganze Zahl ist. Der Wert von n ergibt sich dabei aus den Teilladungen der im Katalysator enthaltenen Elemente. Bei [PMoₓV_{y}O₄₀]ⁿ⁻ beispielsweise ist 3 < n < 10. Als gut geeignet hat sich das Polyoxometallat-Ion [PMoₓV_{y}O₄₀]ⁿ⁻, insbesondere [PMo₇V₅O₄₀]⁸⁻ (HPA-5), erwiesen. Wegen der von den Ionen gebildeten spezifischen Strukturen wird [PMoₓV_{y}O₄₀]ⁿ⁻ auch als Keggin-Ion, [WₓV_{y}O_{19]}ⁿ⁻ als Lindqvist-Ion und [P₂WₓV_{y}O₆₂]ⁿ⁻ als Wells-Dawson-Ion bezeichnet.

Die Oxidation mittels dem molekularen Sauerstoff kann mittels dem molekularen Sauerstoff als reinem Gas oder in einem den molekularen Sauerstoff enthaltenden Gasgemisch, insbesondere Luft oder synthetische Luft, erfolgen. Bei synthetischer Luft handelt es sich im Allgemeinen um ein aus Sauerstoff und Stickstoff bestehendes Gasgemisch, bei welchem der Sauerstoffanteil im Bereich von 19,5 Vol.-% bis 21,5 Vol.-% liegt.

Das den Sauerstoff enthaltende Oxidationsmittel kann ein Peroxid, insbesondere H₂O₂, oder N₂O, sein. Die Oxidation mittels dem molekularen Sauerstoff kann bei einem Sauerstoffdruck - im Falle von Sauerstoff als reinem Gas - oder einem Sauerstoffpartialdruck - im Falle eines Gasgemischs - im Bereich von 1 bar bis 250 bar, insbesondere 1 bar bis 120 bar, insbesondere 1 bar bis 80 bar, insbesondere 1 bar bis 50 bar, insbesondere 1 bar bis 30 bar, insbesondere 5 bar bis 20 bar, insbesondere 5 bar bis 10 bar, erfolgen. Zur Oxidation kann die Lösung, beispielsweise in einem statischen Mischer oder durch heftiges Rühren, mit dem molekularen Sauerstoff beaufschlagt werden.

Die Reaktion zur Erzeugung von Oxymethylenether kann durch eine Erhöhung der Temperatur beschleunigt werden. Bei einer Ausgestaltung des Verfahrens wird die katalytische Reaktion bei einer Temperatur von höchstens 150 °C, insbesondere in einem Bereich von 70 °C bis 150 °C, durchgeführt. Um möglichst wenig des Nebenprodukts Dimethylether im Verhältnis zum gewünschten Oxymethylenether entstehen zu lassen, hat es sich als günstig erwiesen, die katalytische Reaktion bei einer Temperatur im Bereich von 70 °C bis 90 °C durchzuführen.

Für das Verfahren ist es günstig, wenn die Lösung, insbesondere am Beginn der katalytischen Reaktion, möglichst wenig Wasser, insbesondere weniger als 5 Gew.-% Wasser, insbesondere weniger als 1 Gew.-% Wasser, enthält. Bei der katalytischen Reaktion kann/können das Methanol und/oder das Formaldehyd und/oder das Methylformiat als, insbesondere einziges, Lösungsmittel bzw. Lösungsmittelgemisch in der Lösung eingesetzt werden. Das Methanol, das Formaldehyd und/oder das Methylformiat wäre(n) dann gleichzeitig Edukt(e) und Lösungsmittel. Anfänglich können in der mit Sauerstoff beaufschlagten Lösung also lediglich Methanol, das Formaldehyd und/oder das Methylformiat und der Katalysator enthalten sein. Bei einer Ausgestaltung des Verfahrens wird bei der katalytischen Reaktion neben dem molekularen Sauerstoff oder dem Sauerstoff enthaltenden Oxidationsmittel, insbesondere nur, das Methanol als Edukt und, insbesondere einziges, Lösungsmittel in der Lösung eingesetzt.

Bei einer Ausgestaltung des Verfahrens ist die Kettenlänge des zu erzeugenden Oxymethylenethers so gewählt, dass 1 ≤ m ≤ 6 ist. Bei dem Oxymethylenether kann es sich um Dimethoxymethan handeln. In diesem Fall ist m = 1. Dimethoxymethan ist der bei dem Verfahren zuerst entstehende Oxymethylenether. Eine durch den Katalysator bewirkte weitere Umsetzung des Dimethoxymethans kann unterbunden werden, indem das Dimethoxymethan und der Katalysator, insbesondere durch Extraktion oder mittels eines Trennverfahrens unter Verwendung einer semipermeablen Membran, voneinander getrennt werden. Dazu kann entweder der Katalysator oder das Dimethoxymethan aus der Lösung entfernt werden. Alternativ kann der Katalysator in seiner Wirkung auch inaktiviert werden. Bei einem Polyoxometallat als Katalysator kann dies beispielsweise dadurch erfolgen, dass die Lösung alkalisch gemacht wird, beispielsweise indem, insbesondere durch Zusatz eines Hydroxids, der pH-Wert auf einen Wert größer als 8, insbesondere einen Wert größer als 10, insbesondere einen Wert größer als 12, insbesondere einen Wert größer als 13,5, insbesondere einen Wert von 14, eingestellt wird. Polyoxometallate sind bei solchen pH-Werten nicht stabil und werden irreversibel inaktiviert.

Das Dimethoxymethan kann aufgrund seines niedrigen Siedepunkts von 42 °C gut durch Destillation aus dem Reaktionsansatz abgesondert werden. Alternativ kann es durch Extraktion oder mittels eines Trennverfahrens unter Verwendung einer semipermeablen Membran von der Lösung abgesondert werden.

Eine Extraktion von Dimethoxymethan kann mittels eines aus der Tabelle auf Seite 9 der DE 10 2014 112 021 A1 bekannten und für die Extraktion von Dimethoxymethan (mit "X" gekennzeichnete Spalte der Tabelle) geeigneten Extraktionsmittels, welches eine Phasenbildung bewirkt (mit "P" gekennzeichnete Spalte der Tabelle), erfolgen. Dabei kann es sich z.B. um Nitrobenzol, Benzol, Dichlormethan, Ölsäuremethylester oder Diesel handeln.

Soll ein Oxymethylenether erzeugt werden, bei dem m größer als 1 ist, kann die Lösung dazu nach einer Bildung von Dimethoxymethan, insbesondere bei einem Sauerstoffpartialdruck unter 1 bar, insbesondere bei Atmosphärendruck, und/oder einer Temperatur unter 70 °C, insbesondere bei einer Temperatur zwischen 20 °C und 35 °C, weiter inkubiert werden, bis m einen zuvor gewählten Wert erreicht hat. Die Analyse der Lösung zur Bestimmung der Kettenlänge des gebildeten Oxymethylenethers kann beispielsweise mittels Gaschromatografie (GC) unter Verwendung entsprechender Vergleichssubstanzen erfolgen. Die Erfinder haben festgestellt, dass die Erzeugung von Oxymethylenether mit einer größeren Kettenlänge als derjenigen von Dimethoxymethan in der Lösung, katalysiert durch den Katalysator, erfolgt und dass dazu weder ein erhöhter Sauerstoffpartialdruck noch eine erhöhte Temperatur erforderlich sind. Obwohl es grundsätzlich zur Erzeugung des Oxymethylenethers, bei dem m größer als 1 ist, nicht erforderlich ist, kann der Lösung zur Beschleunigung der Kettenverlängerung auch vor oder nach der Bildung des Dimethoxymethans Trioxan zugesetzt werden. Die Reaktion kann dann beispielsweise bei 25 °C und Atmosphärendruck erfolgen. Als günstig hat sich eine Reaktion bei einem Druck von 1 bar bis 20 bar, insbesondere 1 bar bis 10 bar, und bei einer Temperatur von 50 °C bis 200 °C, insbesondere 60 °C bis 130 °C, erwiesen. Die Reaktionsdauer kann beispielsweise im Bereich von 20 Minuten bis 120 Minuten liegen.

Bei einer Ausgestaltung des Verfahrens werden in der katalytischen Reaktion ausschließlich der molekulare Sauerstoff oder das Sauerstoff enthaltendes Oxidationsmittel, Trioxan und Methanol, Formaldehyd und/oder Methylformiat in der Lösung als Edukte eingesetzt. Bei einer weiteren Ausgestaltung des Verfahrens werden in der katalytischen Reaktion ausschließlich der molekulare Sauerstoff oder das Sauerstoff enthaltendes Oxidationsmittel, Trioxan und Methanol in der Lösung als Edukte eingesetzt.

Nachfolgend wird die Erfindung anhand von Ausführungsbeispielen näher erläutert.

Fig. 1 zeigt ein Reaktionsschema der erfindungsgemäßen Umsetzung von Methanol.

### 1. Ausführungsbeispiel:

In einem ersten Ausführungsbeispiel wurden entweder 10 g Methanol als Lösungsmittel und Edukt bzw. Substrat eingesetzt oder jeweils 1 mmol Methylformiat (MF) oder Formaldehyd (FAI) als Substrat in 10 g Methanol als Lösungsmittel eingesetzt. Als Katalysator wurde 0,1 mmol des Polyoxometallat-Ions [PMo₇V₅O₄₀]⁸⁻ (= HPA-5) zugesetzt. Die resultierende Lösung wurde für 24 Stunden mit 1000 Umdrehungen pro Minute gerührt, dabei bei einer Temperatur von 90 °C gehalten und Sauerstoff mit einem Sauerstoffpartialdruck von 20 bar ausgesetzt. Die Ergebnisse sind in der nachfolgenden Tabelle zusammengefasst:

| Substrat | w_{H₂O,Reinstoff} / Gew.-% | w_{H₂O,nach} / Gew.-% | DME | FAI | MM | DMM | FA | MF | Y_{CO2/CO} / % |
|---|---|---|---|---|---|---|---|---|---|
| Methanol (MeOH) | 0,05 | 2,1 | x | - | - | x | - | - | -/- |
| Methylformiat (MF) | 0,15 | 1,8 | x | - | - | x | - | x | -/- |
| Formaldehyd (FAI) | > 50 | 2,4 | x | - | - | x | - | - | 0,6/- |
| | w_{H₂O,vor} = 0,5 | | | | | | | | |

Die Spalte w_{H₂O,Reinstoff} gibt den gewichtsprozentualen Anteil an Wasser in dem jeweiligen Substrat an. Die Spalte w_{H₂O,nach} gibt den gewichtsprozentualen Anteil an Wasser in der Lösung nach Ablauf der Reaktion an. w_{H₂O,vor} gibt bei Formaldehyd den gewichtsprozentualen Anteil an Wasser in der Lösung vor der Reaktion an. Das Vorhandensein der einzelnen Reaktionsprodukte wurde mittels Nuklearmagnetischer Resonanzspektroskopie (NMR) bestimmt. Sofern kein Reaktionsprodukt aufgefunden wurde, wurde dies mit einem "-" gekennzeichnet, ansonsten mit einem "x". Dabei haben die Abkürzungen die folgenden Bedeutungen:
- DME:: Dimethylether
- FAI:: Formaldehyd
- MM:: Methoxymethanol
- DMM:: Dimethoxymethan
- FA:: Ameisensäure
- MF:: Methylformiat

Aus der Tabelle ist ersichtlich, dass bei der Verwendung von Methanol als Ausgangsstoff mit hoher Selektivität nur der Oxymethylenether Dimethoxymethan und das Nebenprodukt Dimethylether und kein CO₂ gebildet wird. Auch wenn Formaldehyd oder Methylformiat eingesetzt wird, werden nur der Oxymethylenether Dimethoxymethan und das Nebenprodukt Dimethylether gebildet. Beim Einsatz von Methylformiat war nach Abschluss der Reaktion noch ein Teil des eingesetzten Methylformiats im Ansatz nachweisbar.

### 2. Ausführungsbeispiel:

Für eine alternative Synthese von OMEs mit einer Kettenlänge von 2 bis 6 wurde ein Molverhältnis von Trioxan zu Dimethoxymethan (Methylal) von 0,33 und der Katalysator in einer Menge von 1 Gew.-% in Bezug auf Trioxan verwendet. Die Reaktionen wurden in einem Glaskolben bei Atmosphärendruck und einer Temperatur von 25 °C durchgeführt. Vor dem Versuch wurde sowohl das Reaktionsgefäß als auch das Methylal getrocknet. Nach Zugabe des Katalysators wurde die resultierende Lösung für 60 Minuten bei 800 Umdrehungen pro Minute gerührt. Eine Analyse der Reaktionsprodukte erfolgte mittels eines Gaschromatografen.

### 3. Ausführungsbeispiel:

Für eine weitere alternative Synthese von OMEs mit einer Kettenlänge von 2 bis 6 wurde ein Edelstahlreaktor verwendet. Dieser wurde mit Dimethoxymethan, Trioxan und dem Katalysator, befüllt. Das molare Verhältnis von Dimethoxymethan zu Trioxan wurde zwischen 2,5:1 und 1:2 variiert und es wurde 2 Gew.-% an Katalysator bezogen auf die Ausgangstoffe eingesetzt. Die Reaktionstemperatur wurde in einem Bereich von 70 °C bis 130 °C eingestellt. Die Reaktionsdauer wurde in einem Bereich von 20 Minuten bis 120 Minuten gewählt. Es wurde ein Reaktionsdruck von 10 bar und eine Rührgeschwindigkeit von 300 Umdrehungen pro Minute eingestellt.

Die Ausführungsbeispiele 2 und 3 zeigten, dass ausgehend von dem bei dem erfindungsgemäßen Verfahren gebildeten Dimethoxymethan unter Zusatz von Trioxan Oxymethylenether mit einer Kettenlänge von 2 bis 6, d. h. Oxymethylenether bei denen 2 ≤ m ≤ 6 gemäß der oben angegebenen allgemeinen Formel ist, erhalten werden können.

## Patentansprüche

1. Verfahren zur Erzeugung von Oxymethylenether der allgemeinen Formel CH₃O-(CH₂O)ₘ-CH₃ in einem Flüssigphasenprozess, wobei 1 ≤ m ≤ 10, wobei in einer katalytischen Reaktion molekularer Sauerstoff oder ein Sauerstoff enthaltendes Oxidationsmittel und Methanol, Formaldehyd und/oder Methylformiat in einer Lösung als Edukte eingesetzt und mittels einer Vanadium in der Oxidationsstufe +IV oder +V enthaltenden Vanadium-Sauerstoff-Verbindung oder eines Salzes davon als Katalysator in der Lösung umgesetzt werden, wobei der bei der katalytischen Reaktion reduzierte Katalysator durch Oxidation mittels dem molekularen Sauerstoff oder dem Sauerstoff enthaltenden Oxidationsmittel wieder in seinen Ausgangszustand versetzt wird, wobei der Katalysator ein Polyoxometallat-Ion der allgemeinen Formel [PMoₓV_{y}O₄₀]ⁿ⁻, wobei 6 ≤ x ≤ 11, 1 ≤ y ≤ 6 und x + y = 12, [WₓV_{y}O₁₉]ⁿ⁻, wobei x + y = 6, 3 ≤ x ≤ 5 und 1 ≤ y ≤ 3 oder [P₂WₓV_{y}O₆₂]ⁿ⁻, wobei x + y = 18, 12 ≤ x ≤ 17 und 1 ≤ y ≤ 6, oder ein VO²⁺ enthaltendes Salz oder ein [VO₃]⁻ enthaltendes Salz ist, wobei n, x und y jeweils eine ganze Zahl ist.

2. Verfahren nach Anspruch 1, wobei der in der katalytischen Reaktion erzeugte Oxymethylenether, insbesondere durch eine Extraktion oder mittels eines Trennverfahrens unter Verwendung einer semipermeablen Membran, aus der Lösung abgesondert wird.

3. Verfahren nach einem der vorhergehenden Ansprüche, wobei in der katalytischen Reaktion ausschließlich der molekulare Sauerstoff oder das den Sauerstoff enthaltende Oxidationsmittel und Methanol, Formaldehyd und/oder Methylformiat als Edukte eingesetzt werden.

4. Verfahren nach einem der vorhergehenden Ansprüche, wobei das VO²⁺ enthaltende Salz VOSO₄ ist und das [VO₃]⁻ enthaltende Salz NH₄VO₃ ist.

5. Verfahren nach einem der vorhergehenden Ansprüche, wobei der molekulare Sauerstoff in einem den molekularen Sauerstoff enthaltenden Gasgemisch, insbesondere Luft, enthalten ist und das den Sauerstoff enthaltende Oxidationsmittel ein Peroxid, insbesondere H₂O₂, oder N₂O, ist.

6. Verfahren nach einem der vorhergehenden Ansprüche, wobei die Oxidation mittels dem molekularen Sauerstoff bei einem Sauerstoffdruck oder Sauerstoffpartialdruck im Bereich von 1 bar bis 50 bar, insbesondere 1 bar bis 30 bar, insbesondere 5 bar bis 20 bar, erfolgt.

7. Verfahren nach einem der vorhergehenden Ansprüche, wobei die katalytische Reaktion bei einer Temperatur von höchstens 150 °C, insbesondere in einem Bereich von 70 °C bis 150 °C, insbesondere in einem Bereich von 70 °C bis 90 °C, durchgeführt wird.

8. Verfahren nach einem der vorhergehenden Ansprüche, wobei die Lösung weniger als 5 Gew.-% Wasser, insbesondere weniger als 1 Gew.-% Wasser, enthält.

9. Verfahren nach einem der vorhergehenden Ansprüche, wobei das Methanol, das Formaldehyd und/oder das Methylformiat bei der katalytischen Reaktion auch als, insbesondere einziges, Lösungsmittel oder Lösungsmittelgemisch eingesetzt wird/werden.

10. Verfahren nach einem der vorhergehenden Ansprüche, wobei 1 ≤ m ≤ 6.

11. Verfahren nach einem der vorhergehenden Ansprüche, wobei der Oxymethylenether Dimethoxymethan ist.

12. Verfahren nach Anspruch 11, wobei eine durch den Katalysator bewirkte weitere Umsetzung des Dimethoxymethans unterbunden wird, indem das Dimethoxymethan und der Katalysator, insbesondere durch Extraktion oder mittels eines Trennverfahrens unter Verwendung einer semipermeablen Membran, voneinander getrennt werden.

13. Verfahren nach Anspruch 11 oder 12, wobei das Dimethoxymethan durch Destillation, Extraktion oder mittels eines Trennverfahrens unter Verwendung einer semipermeablen Membran von der Lösung abgesondert wird.

14. Verfahren nach einem der Ansprüche 1 bis 10, wobei ein Oxymethylenether der allgemeinen Formel CH₃O-(CH₂O)ₘ-CH₃ mit m > 1 gebildet wird, indem die Lösung nach einer Bildung von Dimethoxymethan, insbesondere bei einem Sauerstoffpartialdruck unter 1 bar und/oder einer Temperatur unter 70 °C, weiter inkubiert wird, bis m einen gewählten Wert erreicht hat.

15. Verfahren nach Anspruch 14, wobei der Lösung Trioxan zugesetzt wird.

## Claims

1. A method for production of oxymethylene ether of the general formula CH₃O-(CH₂O)ₘ-CH₃ in a liquid phase process, wherein 1 ≤ m ≤ 10, wherein in a catalytic reaction molecular oxygen or an oxygen-containing oxidant and methanol, formaldehyde, and/or methyl formate are used as reactants in a solution and are converted by means of a vanadium-oxygen compound or a salt thereof as catalyst in the solution, which vanadium-oxygen compound contains vanadium in the oxidation stage +IV or +V, wherein the catalyst reduced during the catalytic reaction is restored to its starting state by oxidation by means of the molecular oxygen or the oxygen-containing oxidant, wherein the catalyst is a polyoxometallate ion of the general formula [PMoₓV_{y}O₄₀]ⁿ⁻, wherein 6 ≤ x ≤ 11, 1 ≤ y ≤ 6 and x + y = 12, [WₓV_{y}O₁₉]ⁿ⁻, wherein x + y = 6, 3 ≤ x ≤ 5 and 1 ≤ y ≤ 3 or [P₂WₓV_{y}O₆₂]ⁿ⁻, wherein x + y = 18, 12 ≤ x ≤ 17 and 1 ≤ y ≤ 6, or is a VO²⁺-containing salt or a [VO₃]⁻-containing salt, wherein n, x and y are in each case an integer.

2. The method according to claim 1, wherein the oxymethylene ether produced in the catalytic reaction is separated from the solution, in particular by an extraction or by means of a separation method using a semi-permeable membrane.

3. The method according to any one of the preceding claims, wherein exclusively the molecular oxygen or the oxygen-containing oxidant and methanol, formaldehyde and/or methyl formate are used as reactants in the catalytic reaction.

4. The method according to any one of the preceding claims, wherein the VO²⁺-containing salt is VOSO₄ and the [VO₃]⁻-containing salt is NH₄VO₃.

5. The method according to any one of the preceding claims, wherein the molecular oxygen is contained in a gas mixture containing the molecular oxygen, in particular air, and the oxygen-containing oxidant is a peroxide, in particular H₂O₂, or N₂O.

6. The method according to any one of the preceding claims, wherein the oxidation by means of the molecular oxygen takes place at an oxygen pressure or an oxygen partial pressure in the range of 1 bar to 50 bar, in particular 1 bar to 30 bar, in particular 5 bar to 20 bar.

7. The method according to any one of the preceding claims, wherein the catalytic reaction is carried out at a temperature of at most 150 °C, in particular in a range of 70 °C to 150 °C, in particular in a range of 70 °C to 90 °C.

8. The method according to any one of the preceding claims, wherein the solution contains less than 5% w/w water, in particular less than 1% w/w water.

9. The method according to any one of the preceding claims, wherein the methanol, the formaldehyde and/or the methyl formate is/are also used in the catalytic reaction as, in particular sole, solvent or solvent mixture.

10. The method according to any one of the preceding claims, wherein 1 ≤ m ≤ 6.

11. The method according to any one of the preceding claims, wherein the oxymethylene ether is dimethoxymethane.

12. The method according to claim 11, wherein a further conversion of the dimethoxymethane caused by the catalyst is prevented by separating the dimethoxymethane and the catalyst from each other, in particular by extraction or by means of a separation method using a semi-permeable membrane.

13. The method according to claim 11 or 12, wherein the dimethoxymethane is separated from the solution by distillation, extraction or by means of a separation method using a semi-permeable membrane.

14. The method according to any one of claims 1 to 10, wherein an oxymethylene ether of the general formula CH₃O-(CH₂O)ₘ-CH₃ with m > 1 is formed by further incubating the solution after formation of dimethoxymethane, in particular at an oxygen partial pressure below 1 bar and/or at a temperature below 70 °C, until m has reached a selected value.

15. The method according to claim 14, wherein trioxane is added to the solution.

## Revendications

1. Procédé de génération d'éther d'oxyméthylène de la formule générale CH₃O-(CH₂O)ₘ-CH₃ dans un processus en phase liquide, dans laquelle 1 ≤ m ≤ 10, dans lequel de l'oxygène moléculaire ou un oxydant contenant de l'oxygène et du méthanol, formaldéhyde et/ou formiate de méthyle sont utilisés dans une solution en tant que réactifs dans une réaction catalytique et sont transformés au moyen d'un composé de vanadium-oxygène contenant du vanadium dans l'étage d'oxydation +IV ou +V ou d'un sel de celui-ci en tant que catalyseur dans la solution, dans lequel le catalyseur réduit lors de la réaction catalytique est ramené à son état de départ par oxydation au moyen de l'oxygène moléculaire ou de l'oxydant contenant de l'oxygène, dans lequel le catalyseur est un ion polyoxométallate de la formule générale [PMoₓV_{y}O₄₀]ⁿ⁻, dans laquelle 6 ≤ x ≤ 11, 1 ≤ y ≤ 6 etx + y= 12, [WₓV_{y}O₁₉]ⁿ⁻, dans laquelle x + y = 6, 3 ≤ x ≤ 5 et1 ≤ y ≤ 3 ou [P₂W_{X}V_{y}O₆₂]ⁿ⁻, dans laquelle x + y = 18, 12 ≤ x ≤ 17 et 1 ≤ y ≤ 6, ou un sel contenant VO²⁺ ou un sel contenant [VO₃]⁻, dans laquelle n, x et y sont chacun un nombre entier.

2. Procédé selon la revendication 1, dans lequel l'éther d'oxyméthylène généré dans la réaction catalytique est séparé de la solution notamment par une extraction ou au moyen d'un procédé de séparation en utilisant une membrane semi-perméable.

3. Procédé selon une des revendications précédentes, dans lequel l'oxygène moléculaire ou l'oxydant contenant l'oxygène et du méthanol, formaldéhyde et/ou formiate de méthyle sont exclusivement utilisés en tant que réactifs dans la réaction catalytique.

4. Procédé selon une des revendications précédentes, dans lequel le sel contenant VO²⁺ est VOSO₄ et le sel contenant [VO₃]⁻ est NH₄VO₃.

5. Procédé selon une des revendications précédentes, dans lequel l'oxygène moléculaire est contenu dans un mélange gazeux contenant l'oxygène moléculaire, notamment de l'air, et l'oxydant contenant l'oxygène est un peroxyde, notamment H₂O₂, ou N₂O.

6. Procédé selon une des revendications précédentes, dans lequel l'oxydation s'effectue au moyen de l'oxygène moléculaire à une pression d'oxygène ou pression partielle d'oxygène dans la région de 1 bar à 50 bars, notamment 1 bar à 30 bars, notamment 5 bars à 20 bars.

7. Procédé selon une des revendications précédentes, dans lequel la réaction catalytique est réalisée à une température d'au plus 150 °C, notamment dans une région de 70 °C à 150 °C, notamment dans une région de 70 ° à 90 °C.

8. Procédé selon une des revendications précédentes, dans lequel la solution contient moins de 5 % en poids d'eau, notamment moins de 1 % en poids d'eau.

9. Procédé selon une des revendications précédentes, dans lequel le méthanol, le formaldéhyde et/ou le formiate de méthyle est/sont utilisé(s) lors de la réaction catalytique également en tant que solvant, notamment unique, ou mélange de solvants.

10. Procédé selon une des revendications précédentes, dans lequel 1 ≤ m ≤ 6.

11. Procédé selon une des revendications précédentes, dans lequel l'éther d'oxyméthylène est le diméthoxyméthane.

12. Procédé selon la revendication 11, dans lequel une autre conversion entraînée par le catalyseur du diméthoxyméthane est empêchée en ce que le diméthoxyméthane et le catalyseur sont séparés l'un de l'autre notamment par extraction ou au moyen d'un procédé de séparation en utilisant une membrane semi-perméable.

13. Procédé selon la revendication 11 ou 12, dans lequel le diméthoxyméthane est séparé de la solution par distillation, extraction ou au moyen d'un procédé de séparation en utilisant une membrane semi-perméable.

14. Procédé selon une des revendications 1 à 10, dans lequel un éther d'oxyméthylène de la formule générale CH₃O-(CH₂O)ₘ-CH₃ est formé avec m > 1 en ce que la solution est davantage incubée après une formation de diméthoxyméthane, notamment à une pression partielle d'oxygène en dessous de 1 bar et/ou une température en dessous de 70 °C, jusqu'à ce que m ait atteint une valeur choisie.

15. Procédé selon la revendication 14, dans lequel du trioxane est ajouté à la solution.
